(19) Europäisches Patentamt
European Patent Office
Office européen des brevets

(11) Publication number: **0 100 788**
**B1**

(12) **EUROPEAN PATENT SPECIFICATION**

(45) Date of publication of patent specification: **10.06.87**

(51) Int. Cl.⁴: **C 07 C 43/12,** C 07 C 41/22

(21) Application number: **82107481.2**

(22) Date of filing: **17.08.82**

(54) **Process for preparing 1,1,2-trifluoro-2-chloroethyl difluoromethyl ether.**

(43) Date of publication of application:
**22.02.84 Bulletin 84/08**

(45) Publication of the grant of the patent:
**10.06.87 Bulletin 87/24**

(84) Designated Contracting States:
**DE FR GB**

(56) References cited:
**FR-A-2 242 356**
**US-A-3 469 011**

(73) Proprietor: **Daikin Kogyo Co., Ltd.**
**Shinhankyu Building No 1-12-39, Umeda Kita-ku**
**Osaka-shi Osaka-fu (JP)**

(72) Inventor: **Takamatsu, Shuichi**
**5-10-12, Daiwanishi**
**Kawanishi-shi Hyogo-ken (JP)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1 (DE)**

**EP 0 100 788 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an improved process for preparing 1,1,2-trifluoro-2-chloroethyl difluoromethyl ether by reacting 1,1,2-trifluoro-2-chloroethyl dichloromethyl ether with hydrogen fluoride.

1,1,2-Trifluoro-2-chloroethyl difluoromethyl ether, useful as an anesthetic, has been prepared by fluorinating 1,1,2-trifluoro-2-chloroethyl dichloromethyl ether with hydrogen fluoride in a liquid phase in the presence of an antimony (V) catalyst or a tin (IV) catalyst (cf. Japanese Patent Publication (unexamined) No. 3,606/1973 and its US-counterpart US—A 3 469 011). However, this process is disadvantageous in that the reaction can hardly be carried out continuously. It is also disadvantageous that the catalyst is, after completion of the reaction, to be decomposed with water and removed.

As a result of the extensive study, it has been found that 1,1,2-trifluoro-2-chloroethyl difluoromethyl ether can be prepared in a good yield by reacting in a gaseous phase 1,1,2-trifluoro-2-chloroethyl dichloromethyl ether with hydrogen fluoride.

Accordingly, the present invention provides a process for preparing 1,1,2-trifluoro-2-chloroethyl difluoromethyl ether by reacting 1,1,2-trifluoro-2-chloroethyl dichloromethyl ether with hydrogen fluoride in the presence of a fluorination catalyst, characterised in that the reaction is carried out in a gaseous state at a temperature of from the boiling point of the starting ether to 400°C in the absence of any catalyst or in the presence of a catalyst selected from the group consisting of oxyfluorides of molybdenum and fluorides and oxyfluorides of chromium and thallium.

In the said conventional liquid-phase fluorination process, the use of the catalyst is essential for the practical rate of the reaction. Further, the fluorination is preferably carried out at a temperature of from 0°C to 10°C, but at a higher temperature, a side reaction may proceed. To the contrary, the gas phase fluorination according to the present invention can well proceed even in the absence of any catalyst and proceeds more advantageously in the presence of a catalyst. Further, the fluorination may be carried out not only at a low temperature but also at a higher temperature with a good yield.

The fluorination of the invention can be carried out continuously with a high yield. When a catalyst is used, continuous operation is possible over a long period of time without producing any waste of the catalyst. These make the process of the invention advantageous in the industry.

As stated above, the fluorination of the present invention may be carried out in the presence or absence of a catalyst, and the desired fluorinated product is obtainable in a good yield. Practically and usually, the catalyst is used, whereby the reaction is accelerated.

The catalysts used are oxyfluorides of molybdenum, and fluorides and oxyfluorides of chromium and thallium. These may be used as such or deposited on a carrier, such as activated charcoal, alumina, fluorinated alumina, calcium fluoride, sodium fluoride and magnesium fluoride. Strong Lewis acids such as titanium tetrafluoride are not favorable, because a clear tendency to lower the yield is observed.

The reaction temperature is from the boiling point of 1,1,2-trifluoro-2-chloroethyl dichloromethyl ether (about 115°C at atmospheric pressure) to 400°C, particularly from 200°C to 400°C when the catalyst is not used, and from said boiling point to 350°C when the catalyst is used. At too high a reaction temperature, the ether linkage is broken to lower the yield. Thus, the reaction temperature is preferred to be as low as possible in the said range.

The reaction pressure is not critical and preferably from 0.5 to 10 atm.

The molar ratio of the starting ether and hydrogen fluoride is not critical and may be usually from 1:2 to 1:20. The space velocity is also not critical and may be usually from 10 to 10,000 $hr^{-1}$. When, for example, 1,1,2-trifluoro-2-chloroethyl dichloromethyl ether and hydrogen fluoride are reacted in a molar ratio of 1:16 in the absence of the catalyst at 245°C with a space velocity of 31 $hr^{-1}$, the yield is 69.6%. When the same starting materials as above are reacted in the same molar ratio as above in the presence of $MoO_xF_y$ deposited on activated charcoal at 160°C with a space velocity of 474 $hr^{-1}$, the yield is 92.1%.

In the fluorination of the invention, the reaction may proceed according to the following reaction formula:

$$CHCl_2OCF_2CFClH \rightarrow CHClFOCF_2CFClH \rightarrow CHF_2OCF_2CFClH$$

Therefore, an intermediate 1,1,2-trifluoro-2-chloroethyl chlorofluoromethyl ether may be contained in the resulting reaction mixture and can easily be recovered on separation of the desired product 1,1,2-trifluoro-2-chloroethyl difluoromethyl ether from the reaction mixture by distillation. The recovered intermediate may be further fluorinated to produce the desired product, which improves the yield.

The present invention will be hereinafter explained in detail by the following Examples.

Example 1

Molybdenum trioxide (5.5 g) was dissolved in 28% aqueous ammonia (50 g) by heating. To the resulting solution, activated charcoal (43 g, about 100 ml) was added, and then water and ammonia were evaporated off under reduced pressure. The residue was charged into the middle portion of a reaction tube made of Hastelloy C (trade name) having an inner diameter of 1 inch, which was placed in an electric furnace, heated in a stream of nitrogen at 300°C for about 5 hours to remove water and ammonia completely and then treated with anhydrous hydrogen fluoride at 300°C for about 5 hours to produce a $MoO_xF_y$ catalyst.

1,1,2-Trifluoro-2-chloroethyl dichloromethyl ether and anhydrous hydrogen fluoride in a molar ratio of 1:16 were passed through in a preheating mixing tube and then the bed of the catalyst in the reaction tube kept at 160°C with a space velocity of 474 $hr^{-1}$. The gaseous reaction mixture was passed through ice water and trapped with a dry-ice/acetone cooled trap. Gas chromatographic analysis of the trapped reaction mixture showed that the yields of 1,1,2-trifluoro-2-chloroethyl difluoromethyl ether, of 1,1,2-trifluoro-2-chloroethyl chlorofluoromethyl ether and of 1,1,2-trifluoro-2-chloroethyl dichloromethyl ether were respectively 92.1%, 1.8% and 0.2%.

Examples 2 to 6

In the same manner as in Example 1 but using the catalyst, the space velocity and the reaction temperature as shown in table 1, the reaction was carried out. Among the catalysts, $MoO_xF_y$/activated charcoal was the same as prepared in Example 1; $AlF_3$, which was used for comparison, was a commercial available one; and $CrO_xF_y$ and $TlO_xF_y$ were prepared by treating $Cr_2O_3$ or $CrO_3$ and $Tl_2O_3$ with anhydrous hydrogen fluoride for several hours at a temperature of from 200°C to 450°C and of 150°C, respectively.

TABLE 1

| Example | Catalyst | Space velocity ($hr^{-1}$) | Reaction temperature (°C) | Yield (%) | | |
|---|---|---|---|---|---|---|
| | | | | A*1) | B*2) | C*3) |
| 2 | $MoO_xF_y$/Activated Charcoal | 474 | 210 | 90.2 | 2.1 | 0.3 |
| 3 | $CrO_xF_y$ | 474 | 125 | 82.2 | 2.4 | 0.2 |
| 4 | α-$AlF_3$ | 474 | 220 | 26.4 | 38.4 | 14.0 |
| 5 | — | 31 | 245 | 69.6 | 7.3 | 0.4 |
| 6 | $TlO_xF_y$ | 474 | 150 | 78.6 | 5.2 | 5.5 |

Note: *1) 1,1,2-Trifluoro-2-chlorethyl difluoromethyl ether
*2) 1,1,2-Trifluoro-2-chloroethyl chlorofluoromethyl ether
*3) 1,1,2-Trifluoro-2-chloroethyl dichloromethyl ether

**Claims**

1. A process for preparing 1,1,2-trifluoro-2-chloroethyl difluoromethyl ether by reacting 1,1,2-trifluoro-2-chloroethyl dichloromethyl ether with hydrogen fluoride in the presence of a fluorination catalyst, characterised in that the reaction is carried out in a gaseous state at a temperature of from the boiling point of the starting ether to 400°C in the absence of any catalyst or in the presence of a catalyst selected from the group consisting of oxyfluorides of molybdenum, and fluorides and oxyfluorides of chromium and thallium.

2. The process according to claim 1, wherein the reaction is carried out at a temperature of from 200°C to 400°C in the absence of a catalyst.

3. The process according to claim 1, wherein the reaction is carried out at a temperature of from the boiling point of the starting ether to 350°C in the presence of the catalyst.

4. The process according to claim 3, wherein the catalyst is one selected from the group consisting of oxyfluorides of molybdenum, chromium and thallium.

**Patentansprüche**

1. Verfahren zur Herstellung von 1,1,2-Trifluoro-2-chloroethyl-difluoromethylether durch Reaktion von 1,1,2-Trifluoro-2-chloroethyl-dichloromethylether mit Hydrogenfluorid in Gegenwart eines Fluorierungs-Katalysators, dadurch gekennzeichnet, daß die Reaktion in der Gas-phase bei einer Temperatur vom Siedepunkt des Ausgangsethers bis 400°C in Abwesenheit jeglichen Katalysators oder in Gegenwart eines aus der aus den Oxyfluoriden des Molybdäns und den Fluoriden und Oxyfluoriden des Chroms und des Thalliums bestehenden Gruppe ausgewählten Katalysators durchgeführt wird.

2. Verfahren nach Anspruch 1, dadurch gekennzeichnet, daß die Reaktion bei einer Temperatur von 200°C bis 400°C in Abwesenheit eines Katalysators durchgeführt wird.

3. Verfahren nach Anspruch 1, dadurch gekennzeichnet, daß die Reaktion bei einer Temperatur vom Siedepunkt des Ausgangsethers bis 350°C in Gegenwart des Katalysators durchgeführt wird.

4. Verfahren nach Anspruch 3, dadurch gekennzeichnet, daß der Katalysator aus der aus den Oxyfluoriden des Molybdäns, des Chroms und des Thalliums bestehenden Gruppe ausgewählt ist.

**Revendications**

1. Un procédé pour fabriquer l'éther 1,1,2-trifluoro-2-chloroéthyl-difluorométhylique par réaction de l'éther 1,1,2-trifluoro-2-chloroéthyl-dichlorométhylique avec le fluorure d'hydrogène en présence d'un catalyseur de fluoration, caractérisé en ce que la réaction est mise en oeuvre à l'état gazeux à une température comprise entre le point d'ébullition de l'éther de départ et 400°C en l'absence de tout catalyseur ou en présence d'un catalyseur choisi parmi les oxyfluorures de molybdène et les fluorures et oxyfluorures de chrome et de thallium.

2. Le procédé selon la revendication 1, dans lequel la réaction est mise en oeuvre à une température de 200 à 400°C en l'absence de catalyseur.

3. Le procédé selon la revendication 1, dans lequel la réaction est mise en oeuvre à une température comprise entre le point d'ébullition de l'éther de départ et 350°C en présence du catalyseur.

4. Le procédé selon la revendication 3, dans lequel le catalyseur est choisi parmi les oxyfluorures de molybdène, de chrome et de thallium.